# DEMANDE DE BREVET EUROPEEN

(11) **EP 1 671 951 A1**
(43) Date de publication de la demande: **21.06.2006**
(21) Numéro de dépôt: 05292691.2
(22) Date de dépôt: 14.12.2005
(51) Int. Cl.: C07D 213/77, C07D 401/14

(54) **Composés diazoiques dissymétriques à groupement2-pyridinium et bras de liaison cationique ou non, compositions les comprenant, procédé de coloration et dispositif**

(30) Priorité: 15.12.2004 FR 0453006
(71) Demandeur: L'ORÉAL, 75008 Paris (FR)
(72) Inventeur: David, Hervé, 94210 La Varenne Saint Hilaire (FR); Greaves, Andrew, 77144 Montevrain (FR); Daubresse, Nicolas, 78170 La Celles St Cloud (FR)
(74) Mandataire: Fevrier, Murielle Françoise E.

(57) **Abrégé**

La présente invention a pour objet des composés cationiques diazoïques dissymétriques à groupement 2-pyridinium et comprenant un bras de liaison cationique ou non.

L'invention concerne de plus des compositions tinctoriales comprenant dans un milieu approprié pour la teinture des fibres kératiniques, de tels composés à titre de colorant direct, ainsi qu'un procédé de coloration de fibres kératiniques mettant en oeuvre cette composition et un dispositif à plusieurs compartiments.

## Description

La présente invention a pour objet des composés cationiques diazoïques dissymétriques à groupement 2-pyridinium et comprenant un bras de liaison cationique ou non, des compositions tinctoriales comprenant dans un milieu approprié pour la teinture des fibres kératiniques, de tels composés à titre de colorant direct, ainsi qu'un procédé de coloration de fibres kératiniques mettant en oeuvre cette composition et un dispositif à plusieurs compartiments.

Il est connu de teindre les fibres kératiniques et en particulier les fibres kératiniques humaines, telles que les cheveux, avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique azinique ou triarylméthane.
Habituellement, ces colorants sont appliqués sur les fibres, éventuellement en présence d'un agent oxydant si l'on souhaite obtenir un effet simultané d'éclaircissement des fibres. Une fois le temps de pose écoulé, les fibres sont rincées, éventuellement lavées et séchées.
Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur relative faible puissance tinctoriale et de leur relative mauvaise tenue aux lavages ou à la transpiration.
Il est connu dans la demande EP 1377263 de mettre en oeuvre des colorants directs cationiques diazoïques particuliers, comprenant deux groupements hétérocycliques cationiques. Ces composés s'ils représentent une avancée dans le domaine, donnent des résultats tinctoriaux qui restent malgré tout encore perfectibles.

Au sens de la présente invention, et à moins qu'une indication différente ne soit donnée :
- Un radical alkyl(èn)e ou la partie alkyl(èn)e d'un radical est linéaire ou ramifiée,
- Un radical alkyl(èn)e ou la partie alkyl(èn)e d'un radical est dite 'substituée' lorsqu'elle comprend au moins un substituant choisi parmi les groupements :
   . hydroxyle,
   • alcoxy en C₁-C₄, (poly)hydroxyalcoxy en C₂-C₄,
   • amino, amino substitué par un ou deux groupements alkyle identiques ou différents en C₁-C₄ éventuellement porteurs d'au moins un groupement hydroxyle ou alcoxy en C₁-C₂, lesdits radicaux alkyle pouvant former avec l'atome d'azote auquel ils sont rattachés, un hétérocycle comprenant de 5 ou 7 chaînons, saturé ou insaturé, éventuellement aromatique, éventuellement substitué comprenant éventuellement au moins un autre hétéroatome différent ou non de l'azote.
   • Un radical alkylcarbonylamino (R'CO-NR-) dans lequel le radical R est un atome d'hydrogène, un radical alkyle en C₁-C₄
   • Un radical alkylsulfonyle (R-SO₂-) dans lequel le radical R représente un radical alkyle en C₁-C₄.
   • Un radical alkylsulfinyle (R-SO-) dans lequel le radical R représente un radical alkyle en C₁-C₄.
   • Un radical alkylcarbonyle (R-CO-) dans lequel le radical R représente un radical alkyle en C₁-C₄.
- Un radical (hétéro)cyclique, saturé ou non, aromatique ou non, ou la partie (hétéro)cyclique, saturée ou non, aromatique ou non, d'un radical est dite 'substituée' lorsqu'elle comprend au moins un substituant, de préférence porté par un atome de carbone, choisi parmi :
   • un radical alkyle en C₁-C₁₆, de préférence en C₁-C₈, éventuellement substitué ;
   • un atome d'halogène tel que chlore, fluor ou brome ;
   • un groupement hydroxyle ;
   • un radical alcoxy en C₁-C₄ ; un radical (poly)-hydroxyalcoxy en C₂-C₄;
   • un radical amino ;
   • un radical amino substitué par un ou deux radicaux alkyle identiques ou différents en C₁-C₄, éventuellement porteurs d'au moins un groupement hydroxyle ou amino ou (mono- ou di-)alkylamino en C₁-C₄ ou alcoxy en C₁-C₂ ; les deux radicaux alkyle pouvant former avec l'atome d'azote auquel ils sont rattachés, un hétérocycle renfermant 1 à 3 hétéroatomes, de préférence 1 à 2 hétéroatomes, choisis parmi N, O, S, de préférence N, l'hétérocycle comprenant de 5 à 7 chaînons, étant saturé ou insaturé, et aromatique ou non, et éventuellement substitué ;

   ■ un radical alkylcarbonylamino (R'CO-NR-) dans lequel le radical R est un atome d'hydrogène, un radical alkyle en C₁-C₄ et le radical R' est un radical alkyle en C₁-C₂ ;
   ■ un radical aminocarbonyle ((R)₂N-CO-) dans lequel les radicaux R, identiques ou non, représentent un atome d'hydrogène, un radical alkyle en C₁-C₄
   ■ un radical alkylsulfonylamino ou arylsulfonylamino (R'SO₂-NR-) dans lequel le radical R représente un atome d'hydrogène, un radical alkyle en C₁-C₄ et le radical R' représente un radical alkyle en C₁-C₄, un radical phényle ;
   ■ un radical aminosulfonyle ((R)₂N-SO₂-) dans lequel les radicaux R, identiques ou non, représentent un atome d'hydrogène, un radical alkyle en C₁-C₄.

Les composés selon la présente invention sont dits dissymétriques lorsqu'il n'existe pas de plan de symétrie perpendiculaire au bras de liaison L. En d'autres termes, les deux membres de formule de part et d'autre du bras de liaison L, sont différents. Plus précisément, ils sont différents lorsque leurs substituants sont différent de part leurs natures et/ou leurs positions dans la molécule.

Au cas où les divers groupements entrant dans la structure des composés selon l'invention sont substitués, l'homme du métier les choisira de telle sorte que la dissymétrie de la molécule soit respectée.

Le but de la présente invention est de fournir des colorants directs ne présentant pas les inconvénients des colorants directs existants.

La présente invention a donc pour objet des composés diazoïques dissymétriques cationiques représentés par la formule (I) suivante, ses formes résonnantes, ainsi que ses sels d'additions avec un acide et/ou ses solvates : 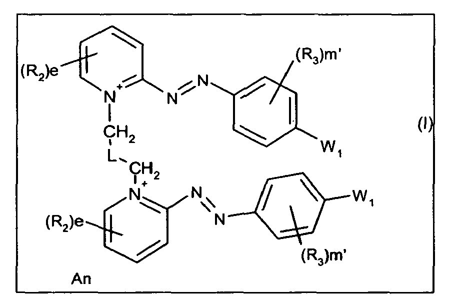 Formules dans lesquelles :
Les radicaux **R**_{**2**}**,** identiques ou non, indépendamment les uns des autres représentent :
   - un radical alkyle en C₁-C₁₆, éventuellement substitué, éventuellement interrompu par un ou plusieurs hétéroatomes et/ou par un ou plusieurs groupements comprenant au moins un hétéroatome, de préférence choisi parmi l'oxygène, l'azote, le soufre, -CO-, -SO₂- ou leurs combinaisons ; ledit radical alkyle étant en outre éventuellement substitué par un ou plusieurs groupements choisis parmi les groupements thio (-SH), thioalkyle en C₁-C₄ ; alkyl(C₁-C₄)sulfinyle, alkyl(C₁-C₄)sulfonyle ;
   - un groupement hydroxyle,
   - un groupement alcoxy en C₁-C₄.
   - un groupement (poly)hydroxyalcoxy en C₂-C₄ ;
   - un groupement alcoxycarbonyle (RO-CO-) dans lequel R représente un radical alkyle en C₁-C₄.
   - un radical alkylcarbonyloxy (RCO-O-) dans lequel R représente un radical alkyle en C₁-C₄.
   - un radical alkylcarbonyle (R-CO-) dans lequel R représente un radical alkyle en C₁-C₄.
   - un groupement amino.
   - un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
   - un groupement alkylcarbonylamino (RCO-NR'-) dans lequel le radical R représente un radical alkyle en C₁-C₄ et le radical R' représente un hydrogène, un radical alkyle en C₁-C₄ ;
   - un groupement aminocarbonyle ((R)₂N-CO-) dans lequel les radicaux R indépendamment les uns des autres, représentent un atome d'hydrogène, un radical alkyle en C₁-C₄ ;
   - un groupement uréido (N(R)₂-CO-NR'-) dans lequel les radicaux R et R', indépendamment les uns des autres, représentent un atome d'hydrogène, un radical alkyle en C₁-C₄ ;
   - un groupement aminosulfonyle ((R)₂N-SO₂-) dans lequel les radicaux R indépendamment les uns des autres, représentent un atome d'hydrogène, un radical alkyle en C₁-C₄ ;
   - un groupement alkylsulfonylamino (RSO₂-NR'-) dans lequel R représente un radical alkyle en C₁-C₄ ; R' représente un atome d'hydrogène, un radical alkyle en C₁-C₄ ;
   - un radical aryle éventuellement substitué ;
   - un radical arylalkyle(C₁-C₄) éventuellement substitué ;
   - un groupement alkylsulfinyle (R-SO-) dans lequel R représente un radical alkyle en C₁-C₄ ;
   - un groupement alkylsulfonyle (R-SO₂-) dans lequel R représente un radical alkyle en C₁-C₄ ;
   - un groupement nitro ;
   - un groupement cyano ;
   - un atome d'halogène, de préférence le chlore, le fluor ;
   - un groupement thio (HS-) ;
   - un groupement alkylthio (RS-) dans lequel le radical R représente un radical alkyle en C₁-C₄ éventuellement substitué ;
   - lorsque e est égal à 2, les deux radicaux R₂ peuvent éventuellement former avec les atomes de carbone auxquels ils sont rattachés, un cycle secondaire, aromatique ou non, à 5 ou 6 chaînons, de préférence à 6 chaînons, éventuellement substitué par un ou plusieurs groupements, identiques ou non, choisis parmi les hydroxyle, alkyle en C₁-C₄, alcoxy en C₁-C₄, (poly)hydroxyalcoxy en C₂-C₄, amino, amino substitué par un ou deux radicaux alkyle identiques ou différents, en C₁-C₄ éventuellement porteurs d'au moins un groupement hydroxyle ;
e est un nombre entier valant de 0 à 4 ; lorsque e est inférieur à 4, le ou les atomes de carbone de l'hétérocycle non substitués issus de la formule (I) porte(nt) un atome d'hydrogène,
Les radicaux **R**_{**3**} identiques ou non, indépendamment les uns des autres représentent :
   - un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, -CO-, -SO₂- ou leurs combinaisons.
   - un groupement hydroxyle,
   - un groupement alcoxy en C₁-C₄.
   - un groupement (poly)hydroxyalcoxy en C₂-C₄ ;
   - un groupement alcoxycarbonyle (RO-CO-) dans lequel R représente un radical alkyle en C₁-C₄.
   - un radical alkylcarbonyloxy (RCO-O-) dans lequel R représente un radical alkyle en C₁-C₄ ;
   - un radical alkylcarbonyle (R-CO-) dans lequel R représente un radical alkyle en C₁-C₄ ;
   - un groupement amino ;
   - un groupement amino substitué par un ou deux radicaux alkyle en C₁-C₄, identiques ou différents, éventuellement porteurs d'au moins un groupement hydroxyle ; les deux radicaux alkyle pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
   - un groupement alkylcarbonylamino (RCO-NR'-) dans lequel le radical R représente un radical alkyle en C₁-C₄ et le radical R' représente un atome d'hydrogène ou un radical alkyle en C₁-C₄ ;
   - un groupement aminocarbonyle ((R)₂N-CO-) dans lequel les radicaux R indépendamment les uns des autres, représentent un atome d'hydrogène, un radical alkyle en C₁-C₄ ;
   - un groupement uréido (N(R)₂-CO-NR'-) dans lequel les radicaux R et R', indépendamment les uns des autres, représentent un atome d'hydrogène, un radical alkyle en C₁-C₄ ;
   - un groupement aminosulfonyle ((R)₂N-SO₂-) dans lequel les radicaux R indépendamment les uns des autres, représentent un atome d'hydrogène, un radical alkyle en C₁-C₄, ;
   - un groupement alkylsulfonylamino (RSO₂-NR'-) dans lequel les radicaux R, R', indépendamment les uns des autres, représentent un atome d'hydrogène, un radical alkyle en C₁-C₄ ;
   - un groupement thio (HS-) ;
   - un groupement alkylthio (RS-) dans lequel le radical R représente un radical alkyle en C₁-C₄ ;
   - un groupement alkylsulfinyle (R-SO-) dans lequel R représente un radical alkyle en C₁-C₄ ;
   - un groupement alkylsulfonyle (R-SO₂-) dans lequel R représente un radical alkyle en C₁-C₄ ;
   - un groupement nitro ;
   - un groupement cyano ;
   - un atome d'halogène, de préférence le chlore, le fluor ;
   - lorsque m' est supérieur ou égal à 2, deux radicaux R₃ adjacents peuvent former avec les atomes de carbone auxquels ils sont rattachés, un cycle secondaire, aromatique ou non à 6 chaînons, éventuellement substitué par un ou plusieurs groupements identiques ou non choisis parmi les groupements hydroxyle, alkyle en C₁-C₄, alcoxy en C₁-C₄, (poly)hydroxyalcoxy en C₂-C₄, alkylcarbonylamino en C₁-C₄, amino, amino substitué par un ou deux radicaux alkyle en C₁-C₄ identiques ou différents éventuellement porteurs d'au moins un groupement hydroxyle.
m' est un nombre entier valant de 0 à 4 ; lorsque m' est inférieur à 4, alors le ou les atomes de carbone du cycle aromatique non substitués issus de la formule (I) porte(nt) un atome d'hydrogène ;
**W**_{**1**} identiques ou non et indépendamment les uns des autres représentent :
   - un atome d'hydrogène.
   - un atome d'halogène choisi parmi le brome, le chlore, le fluor de préférence le chlore et le fluor.
   - un groupement -NR₅R₆, OR₇, -NR₄-Ph-NR₅R₆, -NR₄-Ph-OR₇, -O-Ph-OR₇, -O-Ph-NR₅R₆ ; avec :
      ■ **R**_{**4**}**, R**_{**7**}**,** identiques ou non représentent un atome d'hydrogène, un radical alkyle en C₁-C₂₀, de préférence en C₁-C₁₆ , éventuellement substitué, un radical aralkyle en C₁-C₃ éventuellement substitué, un radical phényle éventuellement substitué ;
      ■ **R**_{**5**} **et R**_{**6**} identiques ou non, représentent un atome d'hydrogène, un radical alkyle en C₁-C₂₀, de préférence en C₁-C₁₆ , éventuellement substitué, un radical phényle éventuellement substitué, un radical aralkyle en C₁-C₃ éventuellement substitué, un radical alkylcarbonyle (R-CO-) dans lequel R est un radical alkyle en C₁-C₄ ;
      ■ **R**_{**5**} **et R**_{**6**} pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
      . **R**_{**5**} et **R**_{**6**} pouvant éventuellement former avec l'atome de carbone du cycle aromatique adjacent à celui auquel -NR₅R₆, est rattaché, un hétérocycle saturé à 5 ou 6 chaînons ;
      ■ **Ph** représentant un radical phényle éventuellement substitué ;
L est un bras de liaison cationique ou non ;
l'électroneutralité du composé de formule (I) étant assurée par un ou plusieurs anions An cosmétiquement acceptables, identiques ou non.

La présente invention a de même pour objet des compositions tinctoriales comprenant, dans un milieu approprié pour la teinture des fibres kératiniques, de tels composés de structure (I) ou ses sels d'addition avec un acide, à titre de colorants directs.
Elle concerne de plus un procédé de coloration de fibres kératiniques consistant à mettre en contact une composition selon l'invention avec lesdites fibres, sèches ou humides, pendant une durée suffisante pour obtenir l'effet souhaité.
Elle a enfin pour objet un dispositif à plusieurs compartiments comprenant, dans un premier compartiment, la composition selon l'invention, et dans un second compartiment, une composition oxydante.

On a constaté que les composés de formule (I) tels que définis précédemment, présentaient une bonne ténacité vis-à-vis d'agents extérieurs comme notamment les shampooings, et cela, même lorsque la fibre kératinique est sensibilisée. Par ailleurs, ces composés présentent des propriétés tinctoriales améliorées telles que la chromaticité, la puissance de coloration ainsi qu'une faible sélectivité, c'est-à-dire que les composés de l'invention permettent d'obtenir des colorations plus uniformes entre la pointe et la racine des cheveux.

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine.

Comme indiqué auparavant, un premier objet de l'invention consiste en des composés correspondant aux formules (I) précitées.

De préférence, les composés de formule (I) selon la présente invention sont tels que les radicaux **R**_{**2**}**,** identiques ou non, indépendamment les uns des autres représentent :
- un atome d'halogène choisi parmi le chlore ou le fluor ;
- un radical alkyle en C₁-C₄, éventuellement substitué par un ou plusieurs radicaux identiques ou différents choisis parmi les radicaux hydroxyle, alcoxy en C₁-C₂, (poly)-hydroxyalcoxy en C₂-C₄, amino, (di)alkylamino en C₁-C₂, thio (-SH), alkyl(C₁₋C₄)sulfinyle, alkyl(C₁-C₄)sulfonyle, thioalkyle(C₁-C₄) ;
- un radical phényle éventuellement substitué par un ou plusieurs radicaux identiques ou différents choisis parmi les radicaux hydroxyle, alcoxy en C₁-C₂, (poly)-hydroxyalcoxy en C₂-C₄, amino, (di)alkylamino en C₁-C₂, ou un atome d'halogène tel que le chlore, le fluor ;
- un radical alcoxy en C₁-C₄ ;
- un radical alkyl(C₁-C₄)sulfonylamino ;
- un radical (poly)-hydroxyalcoxy en C₂-C₄ ;
- un radical amino ;
- un radical (di)alkylamino en C₁-C₂ ;
- un radical (poly)-hydroxyalkylamino en C₂-C₄ ;
- un radical alkylsulfonylamino (RSO₂N-) dans lequel le radical R représente un radical alkyle en C₁-C₄ ;
- un radical aminosulfonyle ((R)₂NSO₂-) dans lequel les radicaux R indépendamment les uns des autres représentent un atome d'hydrogène, un radical alkyle en C₁-C₄ ;
- un radical alkylthio (RS-) dans lequel le radical R représente un radical alkyle en C₁-C₄.
- un radical alkylsulfinyle (RSO-) dans lequel le radical R représente un radical alkyle en C₁-C₄.
- un radical alkylsulfonyle (R-SO₂-) dans lequel le radical R représente un radical alkyle en C₁-C₄ ;
- un radical alkylcarbonylamino (RCONR'-) dans lequel le radical R représente un atome d'hydrogène, un radical alkyle en C₁-C₄ et le radical R' représente un atome d'hydrogène, un radical alkyle en C₁-C₄.

Selon un mode de réalisation particulièrement préféré, les radicaux **R**_{**2**} identiques ou non, indépendamment les uns des autres représentent préférentiellement un radical méthyle, éthyle, 2-hydroxyéthyle, 2-méthoxyéthyle, méthylsulfonyle (CH₃SO₂-), méthylcarbonylamino (CH₃CONH-), hydroxyle, amino, méthylamino, diméthylamino, 2-hydroxyéthylamino, méthoxy, éthoxy, phényle.

Selon une seconde variante préférée, les deux radicaux R₂ issus de la formule (I) peuvent éventuellement former avec les atomes de carbone auxquels ils sont rattachés, un cycle secondaire, aromatique à 6 chaînons, éventuellement substitué par un ou plusieurs groupements identiques ou différents choisis parmi hydroxyle, alkyle en C₁-C₄, alcoxy en C₁₋C₄, amino, amino substitué par un ou deux radicaux alkyle en C₁-C₄ identiques ou différents et éventuellement porteurs d'au moins un groupement hydroxyle ou méthylcarbonylamino.

Conformément à cette seconde variante, les deux radicaux R₂ peuvent éventuellement former avec les atomes de carbone auxquels ils sont rattachés, un cycle secondaire, aromatique à 6 chaînons, éventuellement substitué par un ou plusieurs hydroxyle, méthoxy, éthoxy, amino, 2-hydroxyéthylamino, diméthylamino, (di)-2-hydroxyéthylamino.

Selon un mode réalisation tout particulièrement avantageux, le coefficient e est égal à 0.

Pour ce qui a trait plus particulièrement aux radicaux **R**_{**3**} des formules (I), ces derniers, identiques ou différents, indépendamment les uns des autres représentent plus particulièrement :
- un radical alkyle en C₁-C₁₆, de préférence en C₁-C₈, éventuellement substitué ;
- un atome d'halogène tel que chlore, fluor;
- un groupement hydroxyle ;
- un radical alcoxy en C₁-C₂ ;
- un radical (poly)-hydroxyalcoxy en C₂-C₄ ;
- un radical amino ;
- un radical amino substitué par un ou deux radicaux alkyles en C₁-C₄ identiques ou différents, éventuellement porteurs d'un ou plusieurs groupements identiques ou différents choisi parmi hydroxyle ou alcoxy en C₁-C₄, les deux radicaux alkyle pouvant former avec l'atome d'azote auquel ils sont rattachés, un hétérocycle renfermant 1 à 3 hétéroatomes, de préférence 1 à 2 hétéroatomes, choisis parmi N, O, S, de préférence N, l'hétérocycle comprenant de 5 à 7 chaînons, étant saturé ou insaturé, aromatique ou non, et éventuellement substitué ;
- un radical alkylcarbonylamino (RCO-NR'-) dans lequel le radical R représente un radical alkyle en C₁-C₄ et le radical R' représente un hydrogène, un radical alkyle en C₁-C₄ ;
- un radical alkylsulfonylamino (R'SO₂-NR-) dans lequel le radical R représente un atome d'hydrogène, un radical alkyle en C₁-C₄, le radical R' représente un radical alkyle en C₁-C₄ ;
- un radical aminosulfonyle ((R)₂N-SO₂-) dans lequel les radicaux R identiques ou non, représentent un atome d'hydrogène, un radical alkyle en C₁-C₄ ;
- un radical alkylthio (RS-) dans lequel le radical R représente un radical alkyle en C₁-C₄ ;
- un radical alkylsulfonyle (R-SO₂-) dans lequel le radical R représente un radical alkyle en C₁-C₄.

Plus préférentiellement, lesdits radicaux **R**_{**3**}**,** identiques ou différents, indépendamment les uns des autres, représentent :
- un radical alkyle en C₁-C₄ éventuellement substitué par un ou plusieurs radicaux identiques ou différents choisis parmi les radicaux hydroxyle, alkylcarbonylamino en C₁-C₂, amino substitué par deux radicaux alkyles en C₁-C₂ identiques ou différents éventuellement porteurs d'un ou plusieurs groupements identiques ou différents choisis parmi hydroxyle ou alcoxy en C₁-C₂ ; ces deux radicaux alkyle peuvent éventuellement former avec l'atome d'azote auquel ils sont rattachés, un hétérocycle à 5 ou 6 chaînons, saturé ou non, éventuellement aromatique, choisi de préférence parmi la pyrrolidine, la pipérazine, l'homopipérazine, le pyrrole, l'imidazole, le pyrazole ;
- un radical hydroxyalcoxy en C₂-C₄ ;
- un halogène choisi parmi le chlore ou le fluor ;
- un radical amino ;
- un radical amino substitué par un ou deux radicaux alkyles en C₁-C₂, identiques ou différents éventuellement porteurs d'au moins un groupement hydroxyle;
- un radical méthylcarbonylamino ;
- un radical méthylsulfonylamino ;
- un radical hydroxyle ;
- un radical alcoxy en C₁-C₂ ;
- un radical méthylsulfonyle.

Selon cette variante, les radicaux **R**₃, identiques ou différents, indépendamment les uns des autres, représentent de préférence :
- un radical méthyle, éthyle, propyle, 2-hydroxyéthyle, méthoxy, éthoxy, 2-hydroxyéthyloxy, 3-hydroxypropyloxy, 2-méthoxyéthyle ;
- un radical méthylsulfonylamino ;
- un radical amino, méthylamino, diméthylamino, 2-hydroxyéthylamino.
- un radical méthylcarbonylamino ;
- un radical hydroxyle ;
- un atome de chlore ;
- un radical méthylsulfonyle.

Selon une seconde variante préférée, lorsque le coefficient m' est supérieur ou égal à 2, alors deux radicaux R₃ adjacents peuvent former avec les atomes de carbone auxquels ils sont rattachés, un cycle secondaire aromatique à 6 chaînons, éventuellement substitué par un ou plusieurs groupements identiques ou différents choisis parmi les groupements -NR₄-Ph, -NR₄-Ph-NR₅R₆, -NR₄-Ph-OR₇, hydroxyle, alkyle en C₁-C₄, alcoxy en C₁-C₄, (poly)hydroxyalcoxy en C₂-C₄, alkylcarbonylamino en C₁-C₄, amino, amino substitué par un ou deux radicaux identiques ou différents, alkyle en C₁-C₄, éventuellement porteurs d'au moins un groupement hydroxyle.

Selon cette seconde variante, et de manière encore plus avantageuse, deux radicaux R₃ adjacents peuvent former avec les atomes de carbone auxquels ils sont rattachés, un cycle secondaire aromatique à 6 chaînons, éventuellement substitué par un ou plusieurs groupements choisis parmi les groupements hydroxyle, méthoxy, éthoxy, 2-hydroxyethyloxy, amino, methylcarbonylamino, (di)2-hydroxyethylamino, -NH-Ph, -NH-Ph-NH₂, -NH-Ph-NHCOCH₃, -NH-Ph-OH, -NH-Ph-OCH₃.

Pour ce qui a trait aux radicaux R₄, et R₇, ces derniers représentent :
- un atome d'hydrogène ;
- un radical alkyle en C₁-C₆ éventuellement substitué par un ou plusieurs groupements identiques ou différents de préférence choisis parmi hydroxyle, alcoxy en C₁-C₂;
- un radical aryle, ou arylalkyle, tel que phényle, benzyle, la partie aryle étant éventuellement substituée par un ou plusieurs groupements identiques ou différents de préférence choisis parmi par un atome de chlore, un groupement amino, un groupement hydroxyle, un groupement alcoxy en C₁-C₂, un groupement amino mono ou disubstitué par deux radicaux alkyle en C₁-C₄ identiques ou différents éventuellement porteurs d'au moins un groupement hydroxyle.

Conformément à un mode de réalisation préféré de l'invention, les radicaux R₄, R₇, représentent :
- un atome d'hydrogène ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou différents choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

De préférence, les radicaux R₄, R₇ représentent :
- un atome d'hydrogène ;
- un radical méthyle, éthyle, 2-hydroxyéthyle ;
- un radical phényle, éventuellement substitué par un ou plusieurs radicaux identiques ou différents choisi parmi hydroxyle, méthoxy, amino, (di)méthylamino , (di)(2-hydroxyéthyl)amino.

Pour ce qui a trait aux radicaux R₅, R₆, indépendamment l'un de l'autre, ces derniers identiques ou non, représentent plus particulièrement :
- un atome d'hydrogène ;
- un radical alkylcarbonyle (R-CO-) dans lequel R représente un radical alkyle en C₁₋C₄ éventuellement substitué.
- un radical alkyle en C₁-C₆ éventuellement substitué par un ou plusieurs groupements identiques ou non choisi parmi hydroxyle, alcoxy en C₁-C₂, amino, (di-) alkyle amino en C₁-C₄ ; le radical alkyle peut en outre être substitué par un ou plusieurs groupements identiques ou non choisi parmi alkylsulfonyle en C₁-C₄, alkylsulfinyle en C₁-C₄, alkylcarbonyle en C₁-C₄.
- un radical aryle, ou arylalkyle, tel que phényle, benzyle, la partie aryle étant éventuellement substituée par un ou plusieurs radicaux identiques ou différents choisis parmi chlore, amino, hydroxyle, alcoxy en C₁-C₄, amino mono- ou disubstitué par un ou deux radicaux alkyle en C₁-C₄ identiques ou différents et éventuellement porteurs d'au moins un groupement hydroxyle.

Conformément à un mode de réalisation préféré de l'invention, les radicaux R₅, R₆, identiques ou différents, indépendamment l'un de l'autre représentent avantageusement :
- un atome d'hydrogène ;
- un radical méthylcarbonyle, éthylcarbonyle, propylcarbonyle ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

De manière encore plus préférée, les radicaux R₅, R₆ identiques ou différents, indépendamment l'un de l'autre, représentent :
- un atome d'hydrogène ;
- un radical méthyle, éthyle, 2-hydroxyéthyle ;
- un radical méthylcarbonyle, éthylcarbonyle, propylcarbonyle ;
- un radical phényle, éventuellement substitué par un radical hydroxyle, méthoxy, amino, (di)méthylamino , (di)(2-hydroxyéthyl)amino ;

Il est à noter que selon un mode de réalisation particulier de l'invention, les radicaux R₅ et R₆, forment ensemble avec l'atome d'azote auquel chacun est rattaché, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué.

Avantageusement, l'hétérocycle comprenant de 5 à 7 chaînons est choisi parmi les hétérocycles suivants : pipéridine, 2-(2-hydroxyéthylpipéridine), 4-(aminométhyl)pipéridine, 4-(2-hydroxy éthyl)pipéridine, 4-(diméthylamino)pipéridine, pipérazine, 1-méthylpipérazine, 1-(2-hydroxyéthyl)pipérazine, 1-(2-aminoéthyl)pipérazine, 1-hydroxyéthyléthoxy pipérazine, homopipérazine, 1-méthyl-1,4-perhydrodiazépine, pyrrole, 1,4-diméthylpyrrole, 1-méthyl-4-éthylpyrrole, 1-méthyl-4-propylpyrrole.
De préférence, l'hétérocycle comprenant de 5 à 7 chaînons représente un hétérocycle de type pipéridine, pipérazine, homopipérazine, pyrrole, imidazole, pyrazole éventuellement substitué par un ou plusieurs radicaux identiques ou différents choisis parmi méthyle, hydroxyle, amino, (di)méthylamino.

Selon une troisième variante préférée, les radicaux R₅ et R₆ peuvent former, avec l'atome de carbone du cycle aromatique éventuellement substitué par un hydroxyle et adjacent à celui auquel -NR₅R₆, est rattaché, un hétérocycle saturé à 5 ou 6 chaînons. Par exemple, le groupement -NR₅R₆, avec le noyau aromatique éventuellement substitué par un hydroxyle peut correspondre aux composés suivants :

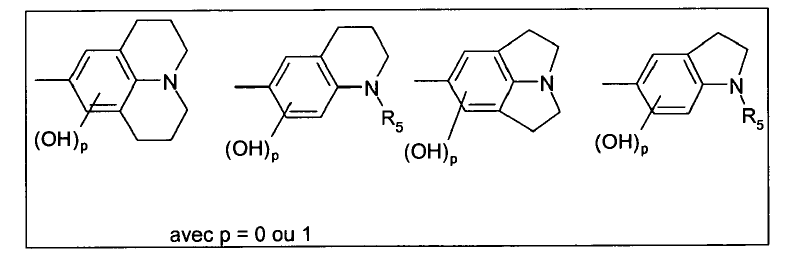

Dans une première variante, L est un bras de liaison non cationique.
Selon cette variante, L bras de liaison non cationique reliant les deux chromophores azoïques différents, représente :
- une liaison covalente ;
- un radical alkyle en C₁-C₄₀, de préférence en C₁-C₂₀, éventuellement substitué, éventuellement interrompu par un (hétéro)cycle saturé ou insaturé, aromatique ou non, comprenant de 3 à 7 chaînons, éventuellement substitué, éventuellement condensé ; le dit radical alkyle étant éventuellement interrompu par un ou plusieurs hétéroatomes ou groupements comprenant au moins un hétéroatome de préférence l'oxygène, l'azote, le soufre, -CO-, -SO₂- ou leurs combinaisons ; le bras de liaison L ne comprenant pas de liaison azo, nitro, nitroso, peroxo ;
- un radical phényle éventuellement substitué.

Selon une seconde variante, le bras de liaison est cationique.
Selon cette variante, L, bras de liaison cationique reliant les deux chromophores azoïques différents, représente :
- un radical alkyle en C₂-C₄₀, portant au moins une charge cationique, éventuellement substitué et/ou éventuellement interrompu par un ou plusieurs (hétéro)cycle saturé ou non, aromatique ou non, identique ou différent, comprenant de 3 à 7 chaînons et/ou éventuellement interrompu par un ou plusieurs hétéroatomes ou groupements comprenant au moins un hétéroatome ou leurs combinaisons, comme par exemple l'oxygène, l'azote, le soufre, un groupement -CO-, -SO₂- ou leurs combinaisons ; le bras de liaison L ne comprenant pas de liaison azo, nitro, nitroso, peroxo ; Etant entendu que le bras de liaison L porte au moins une charge cationique.

Selon une première variante particulière, L représente un bras de liaison non cationique.
Selon cette variante, à titre d'exemple de bras de liaison L de type alkyle préféré, on peut citer les radicaux méthylène, éthylène, propylène linéaire ou ramifié, butylène linéaire ou ramifié, pentylène linéaire ou ramifié, hexylène linéaire ou ramifié, éventuellement substitués et/ou interrompus comme indiqué ci-dessus.
Ces substituants, identiques ou différents sont préférentiellement choisis parmi l'hydroxyle, l'alcoxy en C₁-C₂, le dialkylamino en C₁-C₂, l'alkyl(C₁-C₄)carbonyle, l'alkyl(C₁₋C₄)sulfonyle.

A titre d'exemples préférés de cycle ou d'hétérocycle, saturé ou insaturé, aromatique ou non, interrompant le radical alkyle du bras de liaison L, on peut citer les radicaux phénylène, naphtylène, phénanthrylène, triazinyle, pyrimidinyle, pyridinyle, pyridazinyle, quinoxalinyle, cyclohexyle.

A titre d'exemple de bras de liaison L, on peut citer les radicaux méthylène, éthylène, propylène linéaire ou ramifié, butylène linéaire ou ramifié, pentylène linéaire ou ramifié, hexylène linéaire ou ramifié, éventuellement substitués et/ou interrompus comme indiqué ci-dessus.
A titre d'exemples de cycle ou d'hétérocycle, saturé ou insaturé, aromatique ou non, interrompant le radical alkyle du bras de liaison L, on peut citer les radicaux phénylène ou naphtylène, phénanthrylène, triazinyle, pyrimidinyle, pyridinyle, pyridazinyle, quinoxalinyle, cyclohexyle.

Plus particulièrement, conviennent les radicaux L suivants : 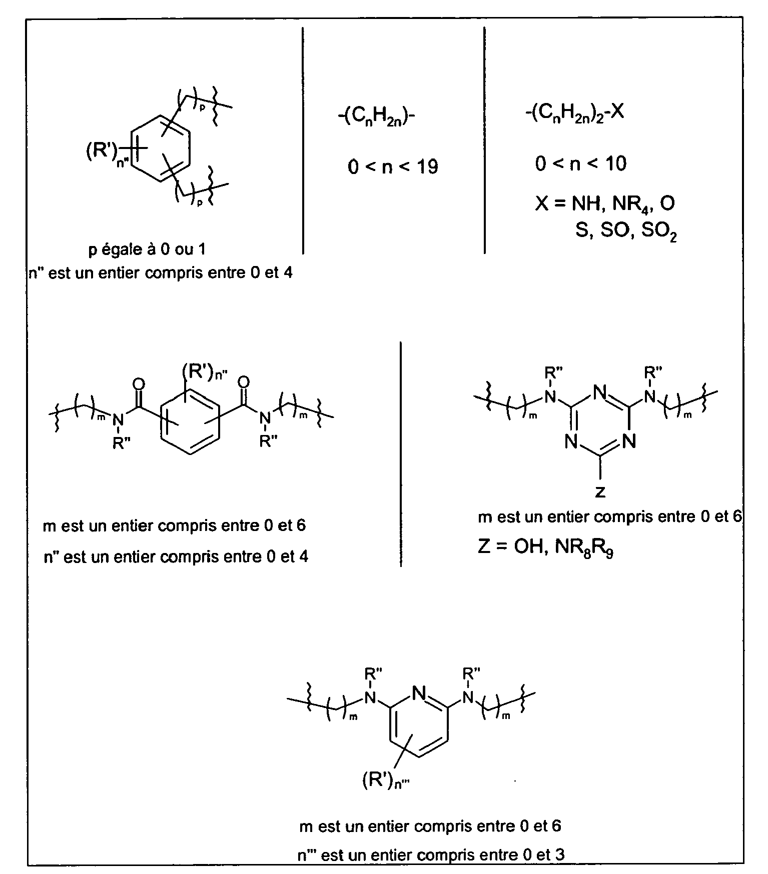 Formules dans lesquelles :
- R' a la même définition que R₃ ;
- R" identiques, représentent un hydrogène, un radical alkyle en C₁-C₄.
- R₈ et R₉ représentent , indépendamment l'un de l'autre, un atome d'hydrogène, un radical alkyle en C₁-C₈ éventuellement substitué par un ou plusieurs radicaux identiques ou différents choisis parmi hydroxyle, alcoxy en C₁-C₂, (poly)hydroxyalcoxy en C₂-C₄, amino, (di-) alkylamino en C₁-C₂ ou aryle éventuellement substitué.

A titre d'exemples de radicaux L préférés, on peut encore citer :

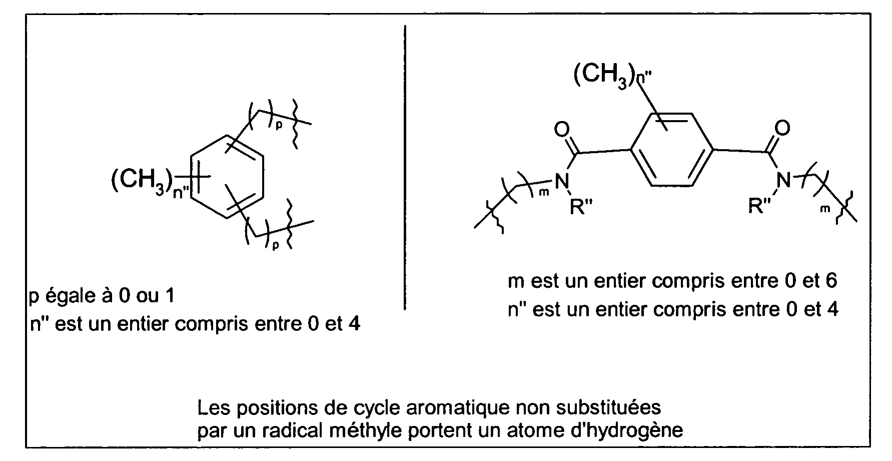

Selon une seconde variante particulière, L représente un bras de liaison cationique.
Selon cette variante, le bras de liaison L cationique, représente avantageusement un radical alkyle en C₂-C₂₀ :
1- interrompu par au moins un groupement correspondant aux formules suivantes : 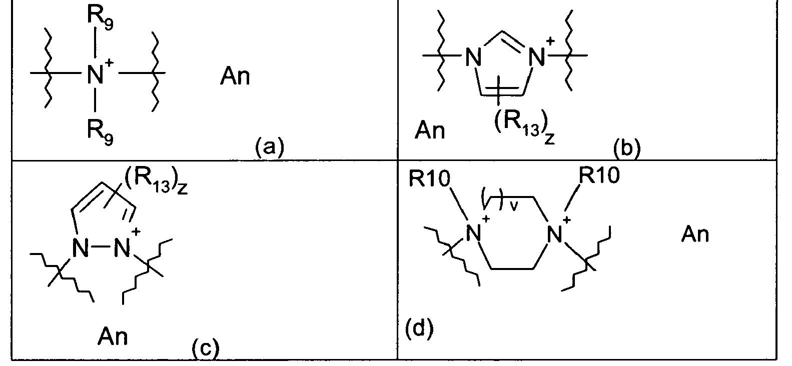 Dans lesquelles :
   - R₉ et R₁₀ indépendamment l'un de l'autre, représentent un radical alkyle en C₁-C₈ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle tel que phényle éventuellement substitué; un radical arylalkyle tel que benzyle éventuellement substitué; un radical aminoalkyle en C₁-C₆ ; un radical aminoalkyle en C₁-C₆ dont l'amine est substituée par un ou deux radicaux alkyle C₁-C₄ identiques ou différents, un radical alkyl(C₁-C₆)sulfonyle.
   - deux radicaux R₉ peuvent former ensemble, avec l'atome d'azote auxquels ils sont rattachés, un cycle saturé ou insaturé, éventuellement substitué, à 5, 6 ou 7 chaînons.
   - R₁₃, identiques ou différents, représentent un atome d'halogène choisi parmi le brome, le chlore ou le fluor, un radical alkyle en C₁-C₆, un radical monohydroxyalkyle en C₁-C₆, un radical polyhydroxyalkyle en C₂-C₆, un radical alcoxy en C₁-C₆, un radical (di-)alkylamino en C₁-C₄, un radical hydroxycarbonyle, un radical alkylcarbonyle en C₁-C₆, un radical thioalkyle en C₁-C₆, un radical alkyl(C₁-C₆)thio, un radical alkyl(C₁-C₆)sulfonyle, un radical benzyle éventuellement substitué, un radical phényle éventuellement substitué par un ou plusieurs radicaux choisis parmi les radicaux méthyle, hydroxyle, amino, méthoxy.
   - An est un anion ou un mélange d'anions, organiques ou minéraux
   - z est un nombre entier compris entre 1 et 3 ; si z < 3, alors les atomes de carbone non substitués portent un atome d'hydrogène.
   - v est un entier égal à 1 ou 2 de préférence égal à 1.
2- Eventuellement interrompu par un ou plusieurs hétéroatomes ou groupements comprenant au moins un hétéroatome ou leurs combinaisons, comme par exemple l'oxygène, l'azote, le soufre, un groupement -CO-, un groupement -SO₂- ; à la condition qu'il n'y ait pas de groupe ou liaison nitro, nitroso, peroxo dans le bras de liaison L ;
3- Et éventuellement substitué par un ou plusieurs radicaux choisis parmi les radicaux hydroxyle, alcoxy en C₁-C₂, (poly)-hydroxyalcoxy en C₂-C₄, amino substitué par un ou plusieurs groupements alkyle linéaires ou ramifiés en C₁-C₂ éventuellement porteurs d'au moins un groupement hydroxyle.

Selon un mode de réalisation particulier des formules (a) et (d), R₉, R₁₀, séparément, sont de préférence choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₂-C₄, un radical diméthylaminoalkyle en C₂-C₆.
De façon encore plus préférée, R₉ et R₁₀ séparément représentent un radical méthyle, éthyle, 2-hydroxyéthyle.

Selon un mode de réalisation particulier des formules (b) et (c), R₁₃ représente un atome d'halogène choisi parmi le chlore et le fluor, un radical alkyle en C₁-C₆, un radical monohydroxylalkyle en C₁-C₄, un radical alkoxy en C₁-C₄, un radical hydroxycarbonyl, un radical alkyl(C₁-C₆)thio, un radical amino disubstitué par un radical alkyle (C₁-C₄).

Selon un mode de réalisation encore plus particulier des formules (b) et (c), R₁₃ représente un atome de chlore, un méthyle, un éthyle, un 2-hydroxyéthyle, un methoxy, un hydroxycarbonyl, un dimethylamino.

Selon un autre mode de réalisation encore plus particulier des formules (b) et (c), z est égal à 0.

Dans la formule (I), An représente un anion ou un mélange d'anions, organiques ou minéraux permettant d'équilibrer la ou les charges des composés de formule (I) par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate ou éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles tel que le mésylate.

Les sels d'addition avec un acide des composés de formule (I) peuvent être à titre d'exemple les sels d'addition avec un acide organique ou minéral tel que l'acide chlorhydrique, l'acide bromhydrique, l'acide sulfurique ou les acides (alkyl- ou phényl-) sulfoniques tels que l'acide p-toluènesulfonique ou l'acide méthylsulfonique.

Les solvates des composés de formule (I) représentent les hydrates de tels composés et l'association d'un composé de formule (I) avec un alcool linéaire ou ramifié en C₁-C₄ tels que le méthanol, l'éthanol, l'isopropanol, le n-propanol.

Conformément à un mode de réalisation préféré de l'invention, les composés correspondent aux formules (I'), (I''), (I''') suivantes, ainsi qu'à leurs formes résonnantes et/ou leurs sels d'addition avec un acide et/ou leurs solvates : 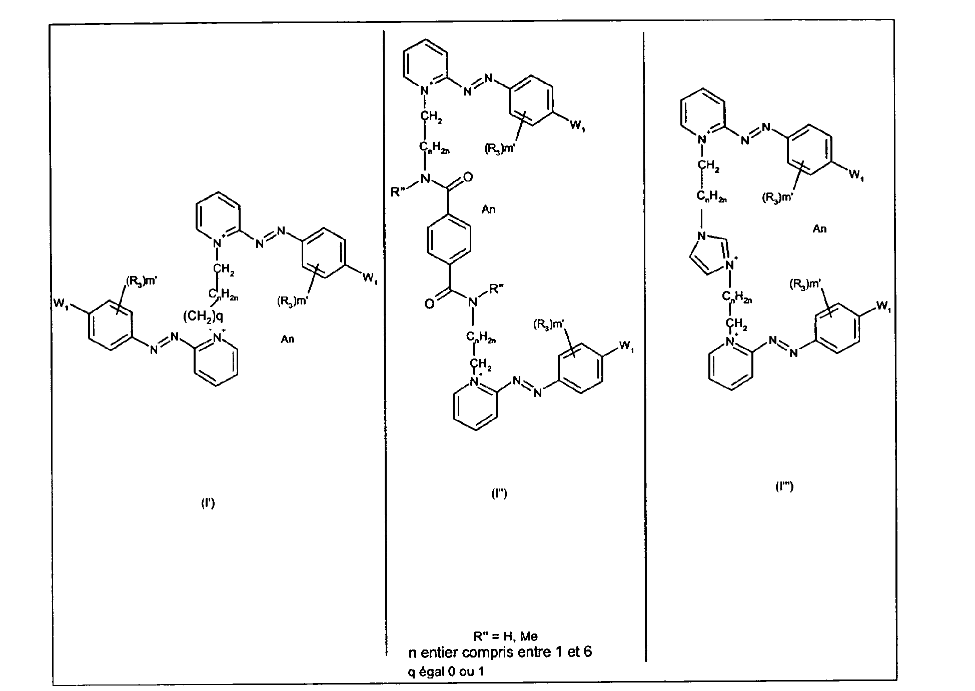 Avec R₃, W₁, m' tels que définis précédemment.

Les composés correspondant aux espèces monoazoïques peuvent notamment être obtenus à partir des procédés de préparation décrits par exemple dans les documents, US 5708151, J.Chem. Res.., Synop. (1998), (10), 648-649, US3151106, US5852179, Hétérocycles, 1987, 26 (2) 313-317, Synth. Commun 1999, 29(13), 2271-2276, Tetrahedron, 1983, 39 (7), 1091-1101. Quant aux composés diazoïques, on pourra se référer à la demande européenne EP 1377263 pour une description de synthèse.

Un autre objet de la présente invention est constitué par une composition tinctoriale comprenant, dans un milieu approprié pour la teinture des fibres kératiniques, au moins un composé de formule (I) ou ses sels d'addition avec un acide et/ou ses solvates, à titre de colorant direct.
La concentration totale en composé(s) de formule (I) peut varier entre 0,001 et 20 % en poids par rapport au poids total de la composition tinctoriale, plus particulièrement entre 0,01 à 10 % en poids, et de préférence entre 0,05 et 5 % en poids.

La composition tinctoriale selon l'invention peut de plus comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.
Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.
Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.
Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.
Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.
Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.
Parmi les bases hétérocycliques, on peut citer à titre d'exemple, les dérivés pyridiniques, les dérivés pyrimidiniques et les dérivés pyrazoliques.
Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.
Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.
Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition tinctoriale selon l'invention peut contenir de plus un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.
A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition tinctoriale de la présente invention, la ou les bases d'oxydation sont présentes en quantité totale de préférence comprise entre 0,001 à 10 % en poids du poids total de la composition tinctoriale, et plus préférentiellement de 0,005 à 6 % en poids.
Le ou les coupleurs sont en général présents en quantité totale comprise entre 0,001 et 10 % en poids du poids total de la composition tinctoriale et plus préférentiellement de 0,005 à 6 % en poids.
D'une manière générale, les sels d'addition avec un acide utilisables dans le cadre des compositions tinctoriales de l'invention pour les bases d'oxydation et les coupleurs sont notamment choisis parmi ceux listés dans le cadre de la définition des composés de formule (I).

La composition selon l'invention peut éventuellement comprendre au moins un colorant direct additionnel différents des composés de formule (I). Celui-ci peut être choisi parmi les espèces cationiques ou non ioniques.
A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.
Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.
Le colorant direct additionnel peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.
On peut aussi mentionner les colorants directs benzéniques nitrés bleus ou violets, comme par exemple :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 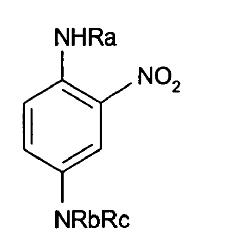
dans laquelle :
- Rb représente un radical alkyle en C₁-C₄, un radical β-hydroxyéthyle ou β-hydroxypropyle ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.
On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.
On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.
Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.
Parmi les colorants aziniques on peut citer les composés suivants :
- Basic Blue 17
- Basic Red 2.
Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7
Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.
Parmi les colorants de type tétraazapentaméthiniques utilisables selon l'invention, on peut citer les composés suivants figurant dans le tableau ci-dessous, An étant défini comme précédemment :

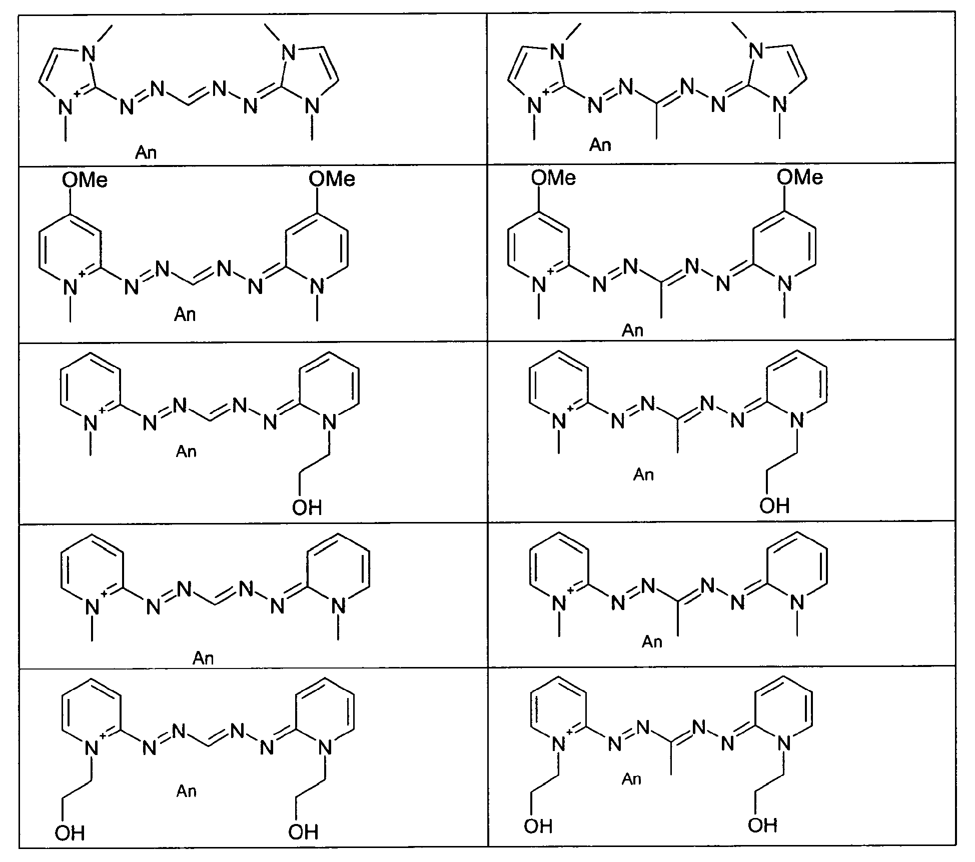

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorant(s) direct(s) additionnels dans la composition varie en général de 0,001 à 20% en poids par rapport au poids de la composition, et de préférence de 0,01 à 10% en poids par rapport au poids de la composition.

Le milieu approprié pour la teinture, appelé aussi support de teinture, est généralement constitué par de l'eau ou par un mélange d'eau et d'au moins un solvant organique pour solubiliser les composés qui ne seraient pas suffisamment solubles dans l'eau.
Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.
Les solvants usuels décrits ci-dessus, s'ils sont présents, représentent habituellement de 1 à 40 % en poids, plus préférentiellement de 5 à 30 % en poids, par rapport au poids total de la composition.

La composition tinctoriale conforme à l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.
Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.
Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 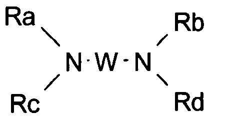 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.
La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La composition selon l'invention peut en outre comprendre au moins un agent oxydant. On parle dans ce cas de composition prête à l'emploi.
Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.
Ladite composition peut aussi être obtenue en mélangeant la composition selon l'invention avec une composition oxydante.
L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.
La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.
Habituellement, on mélange la composition exempte d'agent oxydant avec environ 0,5 à 10 équivalents en poids de la composition oxydante.
Notons que le pH de la composition prête à l'emploi est plus particulièrement compris entre 4 et 12, de préférence entre 7 et 11,5.
Le pH de la composition peut être réglé au moyen d'un agent alcalinisant ou acidifiant, notamment choisis parmi ceux mentionnés auparavant, dans le cadre de la description selon l'invention.

L'invention a aussi pour objet un procédé de coloration qui comprend l'application d'une composition tinctoriale selon l'invention sur les fibres kératiniques, sèches ou humides.
L'application sur les fibres de la composition tinctoriale comprenant le ou les composés de formule (I) ou ses sels d'addition avec un acide, éventuellement au moins une base d'oxydation éventuellement associée à au moins un coupleur, éventuellement au moins un colorant direct additionnel, peut être mise en oeuvre en présence d'agent oxydant.
Cet agent oxydant peut être ajouté à la composition comprenant le ou les composés de formule (I) ainsi que les éventuels base d'oxydation, coupleurs et/ou colorants directs additionnels, soit au moment de l'emploi, soit directement sur la fibre kératinique.
La composition oxydante peut également renfermer divers adjuvants utilisés classiquement dans les compositions pour la teinture des cheveux et tels que définis précédemment.
Le pH de la composition oxydante renfermant l'agent oxydant est tel qu'après mélange avec la composition tinctoriale, le pH de la composition résultante appliquée sur les fibres kératiniques varie de préférence entre 4 et 12 environ, et encore plus préférentiellement entre 7 et 11,5. Il peut être ajusté à la valeur désirée au moyen d'agents acidifiants ou alcalinisants habituellement utilisés en teinture des fibres kératiniques et tels que définis précédemment.
La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Selon un mode de réalisation particulier, la composition selon l'invention est exempte de base d'oxydation et de coupleur.
La composition appliquée peut éventuellement comprendre au moins un agent oxydant.

La composition est donc appliquée sur les fibres kératiniques, sèches ou humides, puis laissée pendant un temps de pose suffisant pour obtenir la coloration recherchée.

Quelle que soit la variante retenue (avec ou sans agent oxydant) le temps de pose est en général compris entre quelques secondes et une heure, de préférence entre 3 et 30 minutes.
La température à laquelle la composition est laissée agir, est en général comprise entre 15 et 220°C, plus particulièrement entre 15 et 80°C, de préférence entre 15 et 40 °C.

A l'issue du temps de pose, la composition est de préférence éliminée par un rinçage à l'eau, suivi éventuellement d'un lavage avec un shampooing, puis éventuellement d'un séchage.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913.

### EXEMPLES

### Synthèse du composé 4 : Synthèse du dibromure de 5-bromo-2-{(E)-[4-(dimethylamino)-2-hydroxyphenyl]diazenyl}-1-[3-(2-{(E)-[4-(dimethylamino)phenyl]diazenyl}pyridinium-1-yl)propyl]pyridinium

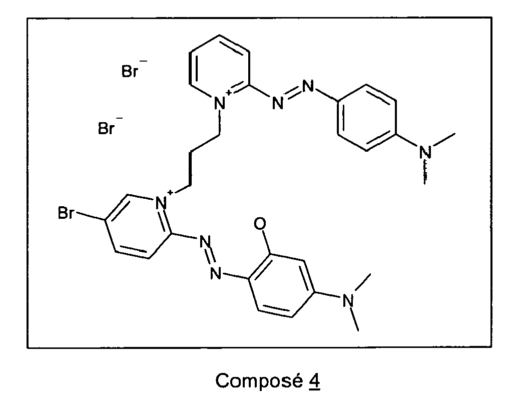

### Etape 1

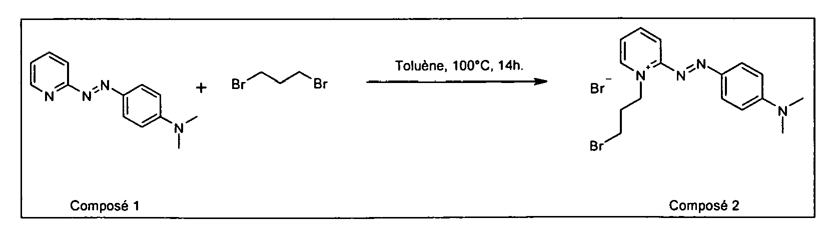

Le composé 1 est un composé commercial

Dans un tricol surmonté d'un réfrigérant, on met sous agitation et à 100 °C pendant 14 heures, le composé 1 (30 g) en présence de 68 ml de 1,3-dibromopropanedans 350 ml de toluène.
Après réaction, le milieu réactionnel est refroidi à température ambiante puis versé sur de l'acétate d'éthyle (500 ml). Le précipité obtenu est filtré puis lavé plusieurs fois à l'acétate d'éthyle et enfin séché sous vide. 44 g d'une poudre violette foncée correspondant au composé 2 sont obtenus.

Les analyses sont conformes au produit attendu.

### Etape 2

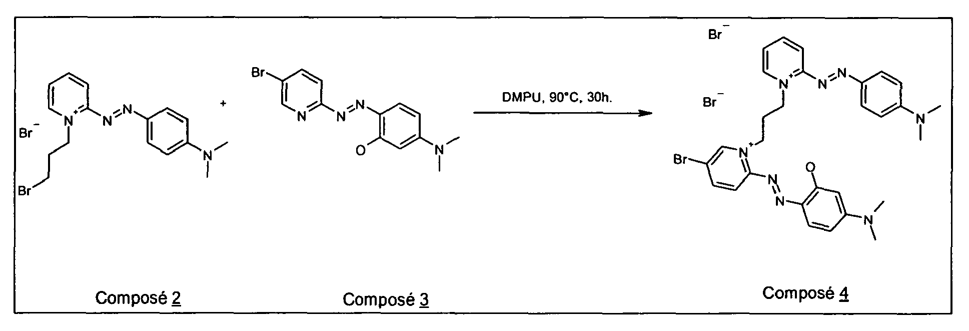

Le composé 3 est un produit commercial.

Dans un tricol surmonté d'un réfrigérant, on met sous agitation et à 90 °C pendant 30 heures, le composé 2 (0.428 g) en présence de 0.64 g de composé 3 dans 10 ml de 1,3-diméthyle-3,4,5,6-tétrahydro-2(1H)-pyrimidinone (DMPU).

Après réaction, le milieu réactionnel est refroidi à température ambiante puis versé sur de l'éther diisopropylique (50 ml). Le précipité obtenu est filtré puis lavé plusieurs fois à l'éther diisopropylique puis à l'acétate d'éthyle et enfin séché sous vide. Le résidu obtenu est purifié par HPLC semi-préparative. Une poudre violette foncée correspondant au composé 4 est obtenu.

Les analyses sont conformes au produit attendu.

### Synthèse du composé 6 : Synthèse du dibromure de 5-bromo-2-{(E)-[4-(diethylamino)-2-hydroxyphenyl]diazenyl}-1-[3-(2-{(E)-[4-(dimethylamino)phenyl]diazenyl}pyridinium-1-yl)propyl]pyridinium

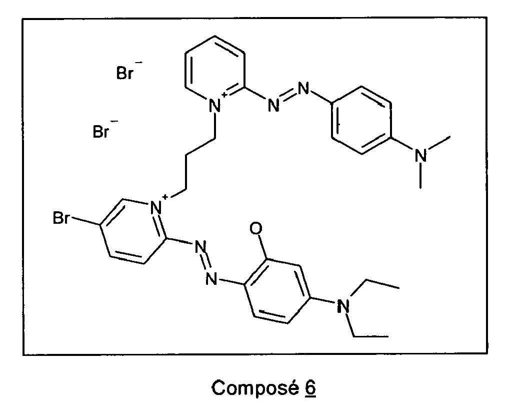

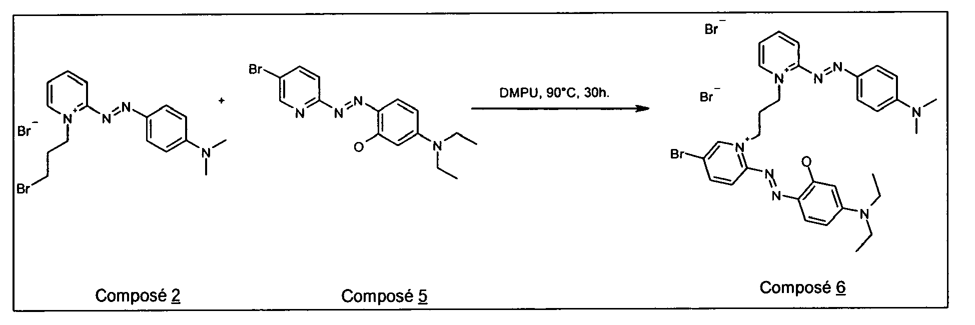

Le composé 5 est un produit commercial.

Dans un tricol surmonté d'un réfrigérant, on met sous agitation et à 90 °C pendant 30 heures, le composé 2 (0.428 g) en présence de 0.70 g de composé 5 dans 10 ml de DMPU.
Après réaction, le milieu réactionnel est refroidi à température ambiante puis versé sur de l'éther diisopropylique (50 ml). Le précipité obtenu est filtré puis lavé plusieurs fois à l'éther diisopropylique puis à l'acétate d'éthyle et enfin séché sous vide. Le résidu obtenu est purifié par HPLC semi-préparative. Une poudre violette foncée correspondant au composé 6 est obtenu.

Les analyses sont conformes au produit attendu.

### Synthèse du composé 8 : Synthèse du dibromure de 2-{(E)-[4-(dimethylamino)phenyl]diazenyl}-1-(3-{2-[(E)-(2-hydroxy-1-naphthyl)diazenyl]pyridinium-1-yl}propyl)pyridinium

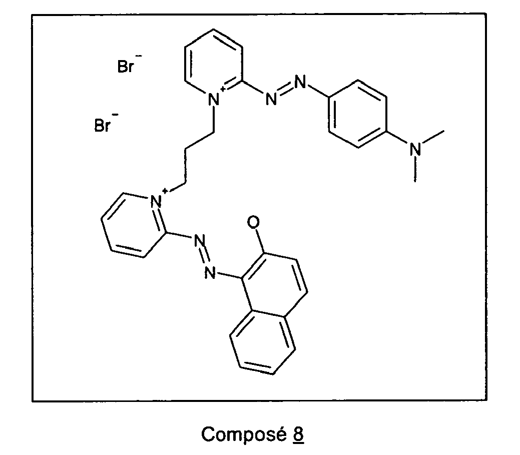

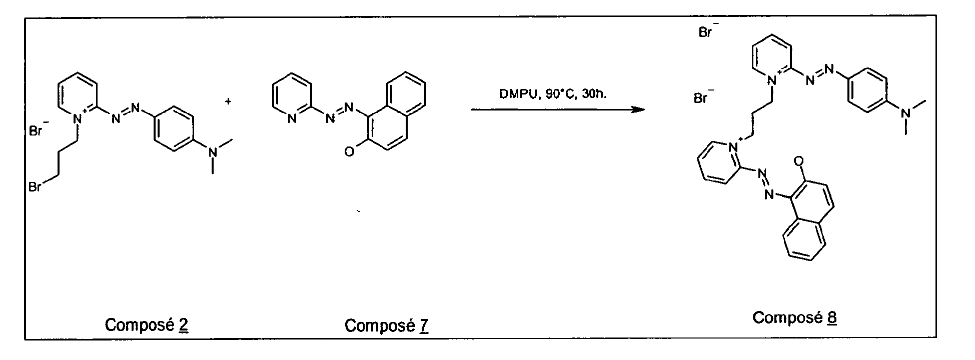

Le composé 7 est un produit commercial.

Dans un tricol surmonté d'un réfrigérant, on met sous agitation et à 90 °C pendant 30 heures, le composé 2 (0.428 g) en présence de 0.50 g de composé 7 dans 10 ml de DMPU.
Après réaction, le milieu réactionnel est refroidi à température ambiante puis versé sur de l'éther diisopropylique (50 ml). Le précipité obtenu est filtré puis lavé plusieurs fois à l'éther diisopropylique puis à l'acétate d'éthyle et enfin séché sous vide. Le résidu obtenu est purifié par HPLC semi-préparative. Une poudre violette foncée correspondant au composé 8 est obtenu.

Les analyses sont conformes au produit attendu.

Lorsque l'on appliquait les colorants décrits ci-dessus sur des cheveux, on obtiendrait les colorations suivantes :

| | |
|---|---|
| **Composé 4** | Violet intense |
| **Composé 6** | Violet intense |
| **Composé 8** | Violet intense |

## Revendications

1. Composés diazoïques dissymétriques cationiques de formule (I) suivante, ses formes résonnantes, ainsi que ses sels d'additions avec un acide et/ou ses solvates : 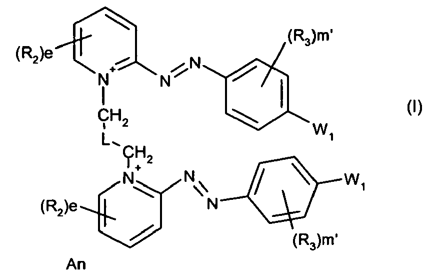 Formules dans lesquelles :
Les radicaux **R**_{**2**}**,** identiques ou non, indépendamment les uns des autres représentent :
• un radical alkyle en C₁-C₁₆, éventuellement substitué, éventuellement interrompu par un ou plusieurs hétéroatomes et/ou par un ou plusieurs groupements comprenant au moins un hétéroatome, de préférence choisi parmi l'oxygène, l'azote, le soufre, -CO-, -SO₂- ou leurs combinaisons ; ledit radical alkyle étant en outre éventuellement substitué par un ou plusieurs groupements choisis parmi les groupements thio (-SH), thioalkyle en C₁-C₄ ; alkyl(C₁-C₄)sulfinyle, alkyl(C₁-C₄)sulfonyle ;
• un groupement hydroxyle,
• un groupement alcoxy en C₁-C₄.
• un groupement (poly)hydroxyalcoxy en C₂-C₄ ;
• un groupement alcoxycarbonyle (RO-CO-) dans lequel R représente un radical alkyle en C₁-C₄.
• un radical alkylcarbonyloxy (RCO-O-) dans lequel R représente un radical alkyle en C₁-C₄.
• un radical alkylcarbonyle (R-CO-) dans lequel R représente un radical alkyle en C₁-C₄.
• un groupement amino.
• un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
• un groupement alkylcarbonylamino (RCO-NR'-) dans lequel le radical R représente un radical alkyle en C₁-C₄ et le radical R' représente un hydrogène, un radical alkyle en C₁-C₄ ;
• un groupement aminocarbonyle ((R)₂N-CO-) dans lequel les radicaux R indépendamment les uns des autres, représentent un atome d'hydrogène, un radical alkyle en C₁-C₄ ;
• un groupement uréido (N(R)₂-CO-NR'-) dans lequel les radicaux R et R', indépendamment les uns des autres, représentent un atome d'hydrogène, un radical alkyle en C₁-C₄ ;
• un groupement aminosulfonyle ((R)₂N-SO₂-) dans lequel les radicaux R indépendamment les uns des autres, représentent un atome d'hydrogène, un radical alkyle en C₁-C₄ ;
• un groupement alkylsulfonylamino (RSO₂-NR'-) dans lequel R représente un radical alkyle en C₁-C₄; R' représente un atome d'hydrogène, un radical alkyle en C₁-C₄ ;
• un radical aryle éventuellement substitué ;
• un radical arylalkyle(C₁-C₄) éventuellement substitué ;
• un groupement alkylsulfinyle (R-SO-) dans lequel R représente un radical alkyle en C₁-C₄ ;
• un groupement alkylsulfonyle (R-SO₂-) dans lequel R représente un radical alkyle en C₁-C₄ ;
• un groupement nitro ;
• un groupement cyano ;
• un atome d'halogène, de préférence le chlore, le fluor ;
• un groupement thio (HS-) ;
• un groupement alkylthio (RS-) dans lequel le radical R représente un radical alkyle en C₁-C₄ éventuellement substitué ;
• lorsque e est égal à 2, les deux radicaux R₂ peuvent éventuellement former avec les atomes de carbone auxquels ils sont rattachés, un cycle secondaire, aromatique ou non, à 5 ou 6 chaînons, de préférence à 6 chaînons, éventuellement substitué par un ou plusieurs groupements, identiques ou non, choisis parmi les hydroxyle, alkyle en C₁-C₄, alcoxy en C₁-C₄, (poly)hydroxyalcoxy en C₂-C₄, amino, amino substitué par un ou deux radicaux alkyle identiques ou différents, en C₁-C₄ éventuellement porteurs d'au moins un groupement hydroxyle ;
e est un nombre entier valant de 0 à 4 ; lorsque e est inférieur à 4, le ou les atomes de carbone de l'hétérocycle non substitués issus de la formule (I) porte(nt) un atome d'hydrogène,
Les radicaux **R**_{**3**} identiques ou non, indépendamment les uns des autres représentent :
• un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, -CO-, -SO₂- ou leurs combinaisons.
• un groupement hydroxyle,
• un groupement alcoxy en C₁-C₄.
• un groupement (poly)hydroxyalcoxy en C₂-C₄ ;
• un groupement alcoxycarbonyle (RO-CO-) dans lequel R représente un radical alkyle en C₁-C₄.
• un radical alkylcarbonyloxy (RCO-O-) dans lequel R représente un radical alkyle en C₁-C₄ ;
• un radical alkylcarbonyle (R-CO-) dans lequel R représente un radical alkyle en C₁-C₄ ;
• un groupement amino ;
• un groupement amino substitué par un ou deux radicaux alkyle en C₁-C₄, identiques ou différents, éventuellement porteurs d'au moins un groupement hydroxyle ; les deux radicaux alkyle pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
• un groupement alkylcarbonylamino (RCO-NR'-) dans lequel le radical R représente un radical alkyle en C₁-C₄ et le radical R' représente un atome d'hydrogène ou un radical alkyle en C₁-C₄ ;
• un groupement aminocarbonyle ((R)₂N-CO-) dans lequel les radicaux R indépendamment les uns des autres, représentent un atome d'hydrogène, un radical alkyle en C₁-C₄ ;
• un groupement uréido (N(R)₂-CO-NR'-) dans lequel les radicaux R et R', indépendamment les uns des autres, représentent un atome d'hydrogène, un radical alkyle en C₁-C₄ ;
• un groupement aminosulfonyle ((R)₂N-SO₂-) dans lequel les radicaux R indépendamment les uns des autres, représentent un atome d'hydrogène, un radical alkyle en C₁-C₄, ;
• un groupement alkylsulfonylamino (RSO₂-NR'-) dans lequel les radicaux R, R', indépendamment les uns des autres, représentent un atome d'hydrogène, un radical alkyle en C₁-C₄ ;
• un groupement thio (HS-) ;
• un groupement alkylthio (RS-) dans lequel le radical R représente un radical alkyle en C₁-C₄ ;
• un groupement alkylsulfinyle (R-SO-) dans lequel R représente un radical alkyle en C₁-C₄ ;
• un groupement alkylsulfonyle (R-SO₂-) dans lequel R représente un radical alkyle en C₁-C₄ ;
• un groupement nitro ;
• un groupement cyano ;
• un atome d'halogène, de préférence le chlore, le fluor ;
• lorsque m' est supérieur ou égal à 2, deux radicaux R₃ adjacents peuvent former avec les atomes de carbone auxquels ils sont rattachés, un cycle secondaire, aromatique ou non à 6 chaînons, éventuellement substitué par un ou plusieurs groupements identiques ou non choisis parmi les groupements hydroxyle, alkyle en C₁-C₄, alcoxy en C₁-C₄, (poly)hydroxyalcoxy en C₂-C₄, alkylcarbonylamino en C₁-C₄, amino, amino substitué par un ou deux radicaux alkyle en C₁-C₄ identiques ou différents éventuellement porteurs d'au moins un groupement hydroxyle.
m' est un nombre entier valant de 0 à 4 ; lorsque m' est inférieur à 4, alors le ou les atomes de carbone du cycle aromatique non substitués issus de la formule (I) porte(nt) un atome d'hydrogène ;
**W**_{**1**} identiques ou non et indépendamment les uns des autres représentent :
• un atome d'hydrogène.
• un atome d'halogène choisi parmi le brome, le chlore, le fluor de préférence le chlore et le fluor.
• un groupement -NR₅R₆, OR₇, -NR₄-Ph-NR₅R₆, -NR₄-Ph-OR₇, -O-Ph-OR₇, -O-Ph-NR₅R₆ ; avec :
■ **R**_{**4**}**, R**_{**7**}**,** identiques ou non représentent un atome d'hydrogène, un radical alkyle en C₁-C₂₀, de préférence en C₁-C₁₆ , éventuellement substitué, un radical aralkyle en C₁-C₃ éventuellement substitué, un radical phényle éventuellement substitué ;
■ **R**_{**5**} **et R**_{**6**} identiques ou non, représentent un atome d'hydrogène, un radical alkyle en C₁-C₂₀, de préférence en C₁-C₁₆ , éventuellement substitué, un radical phényle éventuellement substitué, un radical aralkyle en C₁-C₃ éventuellement substitué, un radical alkylcarbonyle (R-CO-) dans lequel R est un radical alkyle en C₁-C₄ ;
■ **R**_{**5**} **et R**_{**6**} pouvant éventuellement former avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
. **R**_{**5**} et **R**_{**6**} pouvant éventuellement former avec l'atome de carbone du cycle aromatique adjacent à celui auquel -NR₅R₆, est rattaché, un hétérocycle saturé à 5 ou 6 chaînons ;
■ **Ph** représentant un radical phényle éventuellement substitué ;
L est un bras de liaison cationique ou non ;
l'électroneutralité du composé de formule (I) étant assurée par un ou plusieurs anions An cosmétiquement acceptables, identiques ou non.

2. Composés selon la revendication précédente, **caractérisés en ce que** les radicaux R₂, identiques ou non, représentent :
- un atome d'halogène choisi parmi le chlore ou le fluor ;
- un radical alkyle en C₁-C₄, éventuellement substitué par un ou plusieurs radicaux identiques ou différents choisis parmi les radicaux hydroxyle, alcoxy en C₁-C₂, (poly)-hydroxyalcoxy en C₂-C₄, amino, (di)alkylamino en C₁-C₂, thio (-SH), alkyl(C₁₋C₄)sulfinyle, alkyl(C₁-C₄)sulfonyle, thioalkyle(C₁-C₄) ;
- un radical phényle éventuellement substitué par un ou plusieurs radicaux identiques ou différents choisis parmi les radicaux hydroxyle, alcoxy en C₁-C₂, (poly)-hydroxyalcoxy en C₂-C₄, amino, (di)alkylamino en C₁-C₂, ou un atome d'halogène tel que le chlore, le fluor ;
- un radical alcoxy en C₁-C₄ ;
- un radical alkyl(C₁-C₄)sulfonylamino ;
- un radical (poly)-hydroxyalcoxy en C₂-C₄ ;
- un radical amino ;
- un radical (di)alkylamino en C₁-C₂ ;
- un radical (poly)-hydroxyalkylamino en C₂-C₄ ;
- un radical alkylsulfonylamino (RSO₂N-) dans lequel le radical R représente un radical alkyle en C₁-C₄ ;
- un radical aminosulfonyle ((R)₂NSO₂-) dans lequel les radicaux R indépendamment les uns des autres représentent un atome d'hydrogène, un radical alkyle en C₁-C₄ ;
- un radical alkylthio (RS-) dans lequel le radical R représente un radical alkyle en C₁-C₄.
- un radical alkylsulfinyle (RSO-) dans lequel le radical R représente un radical alkyle en C₁-C₄.
- un radical alkylsulfonyle (R-SO₂-) dans lequel le radical R représente un radical alkyle en C₁-C₄ ;
- un radical alkylcarbonylamino (RCONR'-) dans lequel le radical R représente un atome d'hydrogène, un radical alkyle en C₁-C₄ et le radical R' représente un atome d'hydrogène, un radical alkyle en C₁-C₄.

3. Composés selon l'une des revendications précédentes, **caractérisés en ce que** les deux radicaux R₂ issus de la formule (I) peuvent éventuellement former avec les atomes de carbone auxquels ils sont rattachés, un cycle secondaire, aromatique à 6 chaînons, éventuellement substitué par un ou plusieurs groupements identiques ou différents choisis parmi hydroxyle, alkyle en C₁-C₄, alcoxy en C₁-C₄, amino, amino substitué par un ou deux radicaux alkyle en C₁-C₄ identiques ou différents et éventuellement porteurs d'au moins un groupement hydroxyle ou méthylcarbonylamino.

4. Composés selon l'une des revendications précédentes, **caractérisés en ce que** les radicaux R₃, identiques ou différents, représentent :
- un radical alkyle en C₁-C₁₆, de préférence en C₁-C₈, éventuellement substitué ;
- un atome d'halogène tel que chlore, fluor;
- un groupement hydroxyle ;
- un radical alcoxy en C₁-C₂ ;
- un radical (poly)-hydroxyalcoxy en C₂-C₄ ;
- un radical amino ;
- un radical amino substitué par un ou deux radicaux alkyles en C₁-C₄ identiques ou différents, éventuellement porteurs d'un ou plusieurs groupements identiques ou différents choisi parmi hydroxyle ou alcoxy en C₁-C₄, les deux radicaux alkyle pouvant former avec l'atome d'azote auquel ils sont rattachés, un hétérocycle renfermant 1 à 3 hétéroatomes, de préférence 1 à 2 hétéroatomes, choisis parmi N, O, S, de préférence N, l'hétérocycle comprenant de 5 à 7 chaînons, étant saturé ou insaturé, aromatique ou non, et éventuellement substitué ;
- un radical alkylcarbonylamino (RCO-NR'-) dans lequel le radical R représente un radical alkyle en C₁-C₄ et le radical R' représente un hydrogène, un radical alkyle en C₁-C₄ ;
- un radical alkylsulfonylamino (R'SO₂-NR-) dans lequel le radical R représente un atome d'hydrogène, un radical alkyle en C₁-C₄, le radical R' représente un radical alkyle en C₁-C₄ ;
- un radical aminosulfonyle ((R)₂N-SO₂-) dans lequel les radicaux R identiques ou non, représentent un atome d'hydrogène, un radical alkyle en C₁-C₄ ;
- un radical alkylthio (RS-) dans lequel le radical R représente un radical alkyle en C₁-C₄ ;
- un radical alkylsulfonyle (R-SO₂-) dans lequel le radical R représente un radical alkyle en C₁-C₄.

5. Composés selon l'une des revendications précédentes, **caractérisés en ce que** les radicaux R₃, identiques ou différents, représentent :
- un radical alkyle en C₁-C₄ éventuellement substitué par un ou plusieurs radicaux identiques ou différents choisis parmi les radicaux hydroxyle, alkylcarbonylamino en C₁-C₂, amino substitué par deux radicaux alkyles en C₁-C₂ identiques ou différents éventuellement porteurs d'un ou plusieurs groupements identiques ou différents choisis parmi hydroxyle ou alcoxy en C₁-C₂ ; ces deux radicaux alkyle peuvent éventuellement former avec l'atome d'azote auquel ils sont rattachés, un hétérocycle à 5 ou 6 chaînons, saturé ou non, éventuellement aromatique, choisi de préférence parmi la pyrrolidine, la pipérazine, l'homopipérazine, le pyrrole, l'imidazole, le pyrazole ;
- un radical hydroxyalcoxy en C₂-C₄ ;
- un halogène choisi parmi le chlore ou le fluor ;
- un radical amino ;
- un radical amino substitué par un ou deux radicaux alkyles en C₁-C₂, identiques ou différents éventuellement porteurs d'au moins un groupement hydroxyle;
- un radical méthylcarbonylamino ;
- un radical méthylsulfonylamino ;
- un radical hydroxyle ;
- un radical alcoxy en C₁-C₂ ;
- un radical méthylsulfonyle.

6. Composés selon l'une des revendications précédentes, **caractérisés en ce que**, lorsque le coefficient m' est supérieur ou égal à 2, deux radicaux R₃ adjacents peuvent former avec les atomes de carbone auxquels ils sont rattachés, un cycle secondaire aromatique à 6 chaînons, éventuellement substitué par un ou plusieurs groupements identiques ou différents choisis parmi les groupements -NR₄-Ph, -NR₄-Ph-NR₅R₆, -NR₄-Ph-OR₇, hydroxyle, alkyle en C₁-C₄, alcoxy en C₁-C₄, (poly)hydroxyalcoxy en C₂-C₄, alkylcarbonylamino en C₁-C₄, amino, amino substitué par un ou deux radicaux identiques ou différents, alkyle en C₁-C₄, éventuellement porteurs d'au moins un groupement hydroxyle.

7. Composés selon la revendication précédente, **caractérisés en ce que** deux radicaux R₃ adjacents peuvent former avec les atomes de carbone auxquels ils sont rattachés, un cycle secondaire aromatique à 6 chaînons, éventuellement substitué par un ou plusieurs groupements choisis parmi les groupements hydroxyle, méthoxy, éthoxy, 2-hydroxyethyloxy, amino, methylcarbonylamino, (di)2-hydroxyethylamino, -NH-Ph, -NH-Ph-NH₂, -NH-Ph-NHCOCH₃, -NH-Ph-OH, -NH-Ph-OCH₃.

8. Composés selon l'une des revendications précédentes, **caractérisés en ce que** R₄, R₇, indépendamment les uns des autres, représentent :
- un atome d'hydrogène ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou différents choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

9. Composés selon l'une des revendications précédentes, **caractérisés en ce que** les radicaux R₅, R₆, identiques ou différents, représentent :
- un atome d'hydrogène ;
- un radical alkylcarbonyle (R-CO-) dans lequel R représente un radical alkyle en C₁₋C₄ éventuellement substitué.
- un radical alkyle en C₁-C₆ éventuellement substitué par un ou plusieurs groupements identiques ou non choisi parmi hydroxyle, alcoxy en C₁-C₂, amino, (di-) alkyle amino en C₁-C₄; le radical alkyle peut en outre être substitué par un ou plusieurs groupements identiques ou non choisi parmi alkylsulfonyle en C₁-C₄, alkylsulfinyle en C₁-C₄, alkylcarbonyle en C₁-C₄.
- un radical aryle, ou arylalkyle, tel que phényle, benzyle, la partie aryle étant éventuellement substituée par un ou plusieurs radicaux identiques ou différents choisis parmi chlore, amino, hydroxyle, alcoxy en C₁-C₄, amino mono- ou disubstitué par un ou deux radicaux alkyle en C₁-C₄ identiques ou différents et éventuellement porteurs d'au moins un groupement hydroxyle.

10. Composés selon l'une des revendications précédentes, **caractérisés en ce que** les radicaux R₅, R₆, identiques ou différents, représentent :
- un atome d'hydrogène ;
- un radical méthylcarbonyle, éthylcarbonyle, propylcarbonyle ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

11. Composés selon l'une des revendications 1 à 9, **caractérisés en ce que** les radicaux R₅ et R₆, forment ensemble avec l'atome d'azote auquel chacun est rattaché, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué.

12. Composés selon la revendication précédente, **caractérisé en ce que** l'hétérocycle comprenant de 5 à 7 chaînons est choisi parmi les hétérocycles suivants : pipéridine, 2-(2-hydroxyéthylpipéridine), 4-(aminométhyl)pipéridine, 4-(2-hydroxy éthyl)pipéridine, 4-(diméthylamino)pipéridine, pipérazine, 1-méthylpipérazine, 1-(2-hydroxyéthyl)pipérazine, 1-(2-aminoéthyl)pipérazine, 1-hydroxyéthyléthoxy pipérazine, homopipérazine, 1-méthyl-1,4-perhydrodiazépine, pyrrole, 1,4-diméthylpyrrole, 1-méthyl-4-éthylpyrrole, 1-méthyl-4-propylpyrrole.

13. Composés selon l'une des revendications 1 à 8, **caractérisés en ce que** les radicaux R₅ et R₆ peuvent former, avec l'atome de carbone du cycle aromatique éventuellement substitué par un hydroxyle et adjacent à celui auquel -NR₅R₆, est rattaché, un hétérocycle saturé à 5 ou 6 chaînons

14. Composés selon l'une des revendications précédentes, **caractérisés en ce que** L est un bras de liaison non cationique représenté par :
• une liaison covalente ;
• un radical alkyle en C₁-C₄₀, de préférence en C₁-C₂₀, éventuellement substitué, éventuellement interrompu par un (hétéro)cycle saturé ou insaturé, aromatique ou non, comprenant de 3 à 7 chaînons, éventuellement substitué, éventuellement condensé ; le dit radical alkyle étant éventuellement interrompu par un ou plusieurs hétéroatomes ou groupements comprenant au moins un hétéroatome de préférence l'oxygène, l'azote, le soufre, -CO-, -SO₂- ou leurs combinaisons ; le bras de liaison L ne comprenant pas de liaison azo, nitro, nitroso, peroxo ;
• un radical phényle éventuellement substitué.

15. Composés selon l'une des revendications 1 à 13, **caractérisés en ce que** L est un bras de liaison cationique représenté par un radical alkyle en C₂-C₄₀, portant au moins une charge cationique, éventuellement substitué et/ou éventuellement interrompu par un ou plusieurs (hétéro)cycle saturé ou non, aromatique ou non, identique ou différent, comprenant de 3 à 7 chaînons et/ou éventuellement interrompu par un ou plusieurs hétéroatomes ou groupements comprenant au moins un hétéroatome ou leurs combinaisons, comme par exemple l'oxygène, l'azote, le soufre, un groupement -CO-, -SO₂- ou leurs combinaisons ; le bras de liaison L ne comprenant pas de liaison azo, nitro, nitroso, peroxo ; Etant entendu que le bras de liaison L porte au moins une charge cationique.

16. Composés selon l'une quelconque des revendications précédentes, **caractérisés en ce que** An représente un anion ou un mélange d'anions, organiques ou minéraux permettant d'équilibrer la ou les charges des composés de formule (I) choisi parmi un halogénure ; un hydroxyde ; un sulfate ; un hydrogénosulfate ; un alkylsulfate pour lequel la partie alkyle, linéaire ou ramifiée, est en C₁-C₆, les carbonates et hydrogénocarbonates ; des sels d'acides carboxyliques; les alkylsulfonates pour lesquels la partie alkyle, linéaire ou ramifiée, est en C₁-C₆ ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles.

17. Composés selon l'une quelconque des revendications précédentes **caractérisés en ce qu'**ils correspondent aux formules (I'), (I''), (I''') suivantes, ainsi qu'à leurs formes résonnantes et/ou leurs sels d'addition avec un acide et/ou leurs solvates : 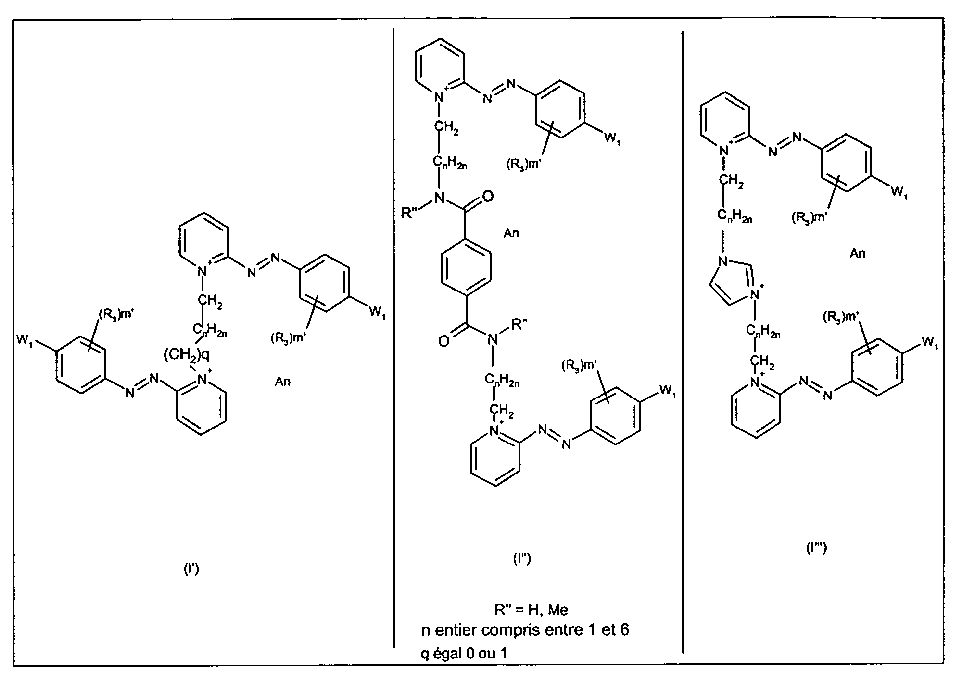 Avec R₃, W₁, m' tels que définis précédemment.

18. Composition tinctoriale comprenant dans un milieu approprié pour la teinture des fibres kératiniques, à titre de colorant direct, au moins un composé de formule (I), ses sels d'addition avec un acide et/ou ses solvates, selon l'une quelconque des revendications précédentes.

19. Composition selon la revendication précédente, **caractérisée en ce que** la teneur en composé de formule (I) ou en chacun des composés de formule (I) varie entre 0,001 et 20 % en poids par rapport au poids total de la composition tinctoriale, plus particulièrement entre 0,01 à 10 % en poids.

20. Composition selon l'une quelconque des revendications 18 ou 19, **caractérisée en ce qu'**elle comprend au moins un colorant direct additionnel, au moins une base d'oxydation éventuellement associée à au moins un coupleur, ou leurs mélanges.

21. Composition selon la revendication précédente, **caractérisée en ce que** le colorant direct additionnel est un colorant cationique ou non ionique, choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

22. Composition selon la revendication 20, **caractérisée en ce que** la base d'oxydation est choisie parmi les p-phénylènediamines, les bis-phénylalkylènediamines, les o-aminophénols, les p-aminophénols, les bases hétérocycliques.

23. Composition selon la revendication 20, **caractérisée en ce que** le coupleur est choisi parmi les m-aminophénols, les m-phénylènediamines, les m-diphénols, les naphtols, les coupleurs hétérocycliques.

24. Composition selon l'une quelconque des revendications 18 à 23, **caractérisée en ce qu'**elle comprend au moins un agent oxydant.

25. Procédé de coloration de fibres kératiniques consistant à mettre en contact une composition selon l'une quelconque des revendications 18 à 24, avec lesdites fibres, sèches ou humides, pendant une durée suffisante pour obtenir l'effet souhaité.

26. Dispositif à plusieurs compartiments dans lequel un premier compartiment renferme la composition selon l'une quelconque des revendications 18 à 23 et un deuxième compartiment renfermant une composition oxydante.
